# EUROPEAN PATENT SPECIFICATION

(11) **EP 1 640 008 B1**
(45) Date of publication and mention of the grant of the patent: **16.02.2011**
(21) Application number: 05019715.1
(22) Date of filing: 09.09.2005
(51) Int. Cl.: A61K 31/522, A61K 9/08

(54) **A stabilized aqueous (6S)-folinic acid preparation for injection**
Wässrige, stabile (6S)-Folinsäurelösung zur Injektion
Préparation aqueuse stable d' acide (6S)-folinique pour injection

(30) Priority: 15.09.2004 JP 2004268489
(43) Date of publication of application: 29.03.2006
(73) Proprietor: NIPRO CORPORATION, Osaka-shi, Osaka 531-8510 (JP)
(72) Inventor: Miyaji, Tatsuaki, Nipro Corporation, Osaka-shi Osaka 531-8510 (JP); Shirouchi, Yutaka, Nipro Corporation, Osaka-shi Osaka 531-8510 (JP); Sato, Makoto, Nipro Corporation, Osaka-shi Osaka 531-8510 (JP); Fujisawa, Yuki, Nipro Corporation, Osaka-shi Osaka 531-8510 (JP)
(74) Representative: Albrecht, Thomas

(56) References cited:
- EP-A- 0 416 232
- EP-A- 0 482 493
- WO-A-2005/080395

## Description

### TECHNICAL FIELD OF THE INVENTION

The present invention relates to a stabilized aqueous preparation for injection containing 5-formyl-(6S)-tetrahydrofolic acid or a pharmacologically acceptable salt thereof as an active ingredient.

### BACKGROUND ART OF THE INVENTION

5-Formyl-(6S)-tetrahydrofolic acid, which is used as an active ingredient in the present invention, is known, and another name for it is (6S)-N-[4-[[(2-amino-5-formyl-1,4,5,6,7,8-hexahydro-4-oxo-6-pteridinyl)methyl]amino]benzoyl]-L-glutamic acid. The compound is also called (6S) -folinic acid and is a reduced folic acid derivative. Its calcium salt, i.e., calciumlevofolinate [trade name: Isovorin (Wyeth-Takeda Pharmaceutical Company Limited)] is on the market as an enhancer for the antitumor effect of fluorouracil on stomach and colon/rectal cancers. 5-Formyltetrahydrofolic acid has optical isomers of the D-form (6R-form) and L-form (6S-form) due to the configuration of the carbon atom at the 6-position. The L-form is known as a medicinally active form, but both the racemic and L-forms have already been marketed [trade name of the racemic form: Leucovorin (Wyeth-Takeda Pharmaceutical Company Limited)]. The racemic form is generally stable, while the L-form is known to be unstable and to undergo hydrolysis and oxidative degradation in an aqueous solution. Therefore, the L-form formulations that have been marketed up to the present time are in the form of freeze-dried preparations.

Although some reports on the stabilization of formulations of 5-formyl- (6S) -tetrahydrofolic acid derivatives have already been published, only unsatisfactory results are obtained in all reports. Prior art documents are listed below. A formulation which is a mixture used as an injection solution including a salt of Leucovorin, tromethamine, and monothioglycerol, and which is stable against sunlight exposure for a long time is described in EP 0416232A and USP 5,173,488. Tromethamine, i.e., 2-amino-2-(hydroxymethyl)-1,3-propanediol, is used as a buffer agent and monothioglycerol is used as an antioxidant. However, the Leucovorin formulation disclosed is in the DL-mixed racemic form, and properties thereof are completely different from those of the L-form. The Merck Index 13^{th} Edition, 4248 (2001) discloses that Leucovorin is a reduced folic acid formulation, and is more stable at a neutral or mildly alkaline pH. It discloses that calcium levofolinate is the L-form and is calcium (6S)-folinate. A composition (racemic) containing 200 to 2,000 mg of a mixture of 95% by weight or more of a (6S)-diastereomer of calcium 5-formyl-tetrahydrofolate and 5% by weight or less of a (6R)-diastereomer of calcium 5-formyl-tetrahydrofolate, and an acceptable carrier for human treatment is described in USP 4, 959,472 and EP 0266042A. A composition containing 5-formyl-(6S)-tetrahydrofolic acid, a food formulation or an essential nutrient formulation is described in WO97/27764. A 5,10-methylene-tetrahydrofolic acid-cyclodextrin inclusion compound is described in USP 5,455,236 and EP 0579996A. In USP 5,817,659 and EP 0773221A, a stable crystalline tetrahydrofolic acid is described. In USP 5,177,076 and EP 0401895A, an aqueous folinic acid solution which is stable under refrigeration, and a production method are described. In USP 4, 350, 659 and GB 2106103A, a method of stabilizing a folic acid derivative is described.

### SUMMARY OF THE INVENTION

5-Formyl-(6S)-tetrahydrofolic acid or its pharmacologically acceptable salt, which enhances an antitumor effect of fluorouracil on stomach and colon/rectal cancers, is unstable in an aqueous solution, and can not be stored for a long period. Therefore, the formulations that have been marketed up to the present are in the form of freeze-dried solid preparations. Development of a stabilized aqueous preparation for injection containing 5-formyl-(6S)-tetrahydrofolic acid or its pharmacologically acceptable salt which can be immediately administered parenterally to a patient has been demanded.

In view of such circumstances, the present inventors have made extensive studies. As a result, they have found that an aqueous preparation containing 5-formyl-(6S)-tetrahydrofolic acid or a pharmacologically acceptable salt thereof showing extremely excellent preservation stability is obtained by adding an antioxidant and a pH regulator to the aqueous preparation. Further, the present inventors continued their studies and found that 5-formyl-(6S)-tetrahydrofolic acid or a pharmacologically acceptable salt thereof is stable in a selective pH value range of the aqueous preparation. Based on these findings and devising its package form, they have completed the present invention.

That is, the present invention relates to:
(1) an aqueous preparation for injection which comprises 5-formyl-(6S)-tetrahydrofolic acid or a pharmacologically acceptable salt thereof as an active ingredient;
(2) the preparation according to item (1), wherein the pharmacologically acceptable salt of 5-formyl-(6S)-tetrahydrofolic acid is an alkali metal salt or an alkaline earth metal salt;
(3) the preparation according to item (1), wherein the pharmacologically acceptable salt of 5-formyl-(6S)-tetrahydrofolic acid is a calcium salt;
(4) the preparation according to item (1), wherein a pH regulator and an antioxidant are contained,
(5) the preparation according to item (4), wherein the antioxidant is selected from the group consisting of ascorbic acid or a salt thereof, sodium pyrosulfite, cysteine hydrochloride, sodium hydrogensulfite, sodium sulfite, thioglycerol, and thioglycolic acid or a salt thereof;
(6) the preparation according to item (4), wherein the antioxidant is ascorbic acid or a salt thereof;
(7) the preparation according to item (4), wherein the antioxidant is a combination of ascorbic acid or a salt thereof with another antioxidant;
(8) the preparation according to item (7), wherein the another antioxidant is selected from the group consisting of sodium pyrosulfite, cysteine hydrochloride, sodium hydrogensulfite, sodium sulfite, thioglycerol, and thioglycolic acid or a salt thereof;
(9) the preparation according to item (4), wherein the antioxidant is present in an amount of 0.005 to 1 w/v%;
(10) the preparation according to item (4), wherein the pH regulator is tromethamine;
(11) the preparation according to item (4), wherein the pH regulator is present in an amount of 0.01 to 1 w/v%;
(12) the preparation according to item (1), wherein a pH value of the aqueous solution is adjusted to 6 to 7;
(13) the preparation according to item (1), wherein a pH value of the aqueous solution is adjusted to 8.5 to 9.0;
(14) a pharmaceutical package comprising the preparation defined in item (1), contained in at least one packaging form selected from:
   1) a glass or plastic container;
   2) an inner container of a double package form, which comprises an inner container and an outer bag, where a deoxidant is included between the inner container and the outer bag;
   3) a container where air in the inside of the container is substituted by an inert gas;
   4) a glass or plastic prefilled syringe;
   5) a prefilled syringe as an inner container of a double package form which comprises an inner container and an outer bag, where a deoxidant is included between the inner container and the outer bag; and
   6) a prefilled syringe where air in the inside of the prefilled syringe is substituted by an inert gas;
(15) a method of stabilizing an aqueous preparation for injection containing 5-formyl-(6S)-tetrahydrofolic acid or a pharmacologically acceptable salt thereof, characterized in that an antioxidant is added to an aqueous solution comprising 5-formyl-(6S)-tetrahydrofolic acid or a pharmacologically acceptable salt thereof as an active ingredient;
(16) the method according to item (15), wherein a pH regulator is further added; and
(17) a pharmaceutical aqueous preparation for injection, which contains calcium levofolinate as an active ingredient, ascorbic acid or a salt thereof, and a pH regulator.

### EFFECTS OF THE INVENTION

In the present invention, an aqueous preparation for injection containing 5-formyl-(6S)-tetrahydrofolic acid or a pharmacologically acceptable salt thereof as an active ingredient (hereinafter referred to as the preparation of the present invention) shows excellent preservation stability. In the case of use of the preparation in the clinical field, it is not necessary to dissolve a solid formulation afresh, and the preparation can be immediately administered parenterally to a patient, or simply by diluting it to an appropriate concentration with a diluent. A lowering of pH value and a lowering of transmittance of the aqueous preparation can sufficiently be controlled. These properties enhance the availability of 5-formyl-(6S)-tetrahydrofolic acid or a pharmacologically acceptable salt thereof.

### DETAILED DESCRIPTION OF THE INVENTION

In the present invention, 5-formyl-(6S)-tetrahydrofolic acid is preferably used in the form of a pharmacologically acceptable salt thereof. Such salt is a conventional nontoxic salt, and examples include a salt with a base, such as a salt with an inorganic base, e.g., an alkali metal salt (such as a sodium salt or a potassium salt) and an alkaline earth metal salt (such as a calcium salt or a magnesium salt). Particularly preferable is a calcium salt. These pharmacologically acceptable salts include an anhydrous salt which is an anhydride and a hydrate salt which is a hydrate. 5-Formyl-(6S)-tetrahydrofolic acid which is an active ingredient of the preparation of the present invention is an L-form optically active compound. It is also referred to as the (6S)-form. In the preparation of the present invention, the content of 5-formyl-(6S)-tetrahydrofolic acid or a pharmacologically acceptable salt thereof is generally 0.01 to 5 w/v%.

A pH regulator and an antioxidant are preferably contained in the preparation of the present invention. The pH regulator and the antioxidant are well-known substances, and widely known pH regulators and antioxidants can be used in the present invention.

Examples of the pH regulator include a basification material and a buffer agent. Examples of the basification material include an inorganic base, an organic base, a basic organic acid, and a metal oxide.

Examples of the inorganic base include an alkali metal hydroxide (for example, sodium hydroxide or potassium hydroxide), and an alkaline earth metal hydroxide (for example, magnesium hydroxide or calcium hydroxide).

Examples of the organic base include tromethamine (tromethamol), succinamide, adamantylamine, N,N'-bis(2-hydroxyethyl)-ethylenediamine, tris(hydroxyethyl)-aminomethane, N-methyl glucamine (meglumine), monoethanolamine, diethanolamine, and triethanolamine.

Examples of the basic organic acid include a basic amino acid (for example, lysine, arginine, histidine, or ornithine), and alkali metal salts of 3-(N-morpholino)propanesulfonic acid, 2-(N-morpholino)- ethanesulfonic acid, 3-[cyclohexylamino]-1-propanesulfonic acid, 4-[cyclohexylamino]-1-propanesulfonic acid and 2-[cyclohexylamino]- 1-ethanesulfonic acid.

Examples of the metal oxide include magnesium oxide and aluminum oxide. Examples of the buffer agent include a salt of an inorganic acid, a salt of an organic acid, a salt of an organic base, and an amino acid.

Examples of the salt of an inorganic acid include sodium phosphate, potassium phosphate, sodium hydrogenphosphate, potassium hydrogenphosphate, dibasic sodium phosphate, sodium carbonate, potassium carbonate, magnesium carbonate, calcium carbonate, sodium bicarbonate, potassium bicarbonate, magnesium bicarbonate, calcium bicarbonate, sodium sulfate, potassium sulfate, magnesium sulfate, and calcium sulfate.

Examples of the salt of an organic acid include potassium salts and sodium salts of acetic acid, citric acid, lactic acid, maleic acid, phenylacetic acid, benzoic acid, and lauryl sulfuric acid.

Examples of the salt of an organic base include: carbonates and bicarbonates of aminoguanidine, guanidine, adamantylamine, N,N'-bis(2-hydroxylethyl)ethylenediamine and tris(hydroxymethyl)aminomethane.

Examples of the amino acid include glycine, alanine, valine, serine, and threonine. Preferable examples of the pH regulator include glycine and tromethamine. Of those, tromethamine is particularly preferable.

The addition amount of the pH regulator is determined by a desired pH value and volume of the solution. An aqueous preparation of the present invention may be pH 5.5 to 7.5, preferably pH 6 to 7, or may be pH 8.3 to 9.0, preferably pH 8.5 to 9.0, and may be more preferably pH 6 to 7. Therefore, the pH regulator is added so as to be contained in a concentration of 0.01 to 1 w/v%, preferably 0.02 to 0.5 w/v%. The pH value of the solution can be controlled by appropriately using an acid (for example, hydrochloric acid) and a base (for example, an aqueous sodium hydroxide solution).

Examples of the antioxidant include ascorbic acid or a salt thereof, sodium pyrosulfite, cysteine hydrochloride, sodium hydrogensulfite, thioglycerol, thioglycolic acid or a salt thereof, and sodium sulfite. Preferably, ascorbic acid or a salt thereof is used, and an example of the salt includes a pharmacologically acceptable salt thereof. Most preferably, ascorbic acid or its sodium salt is used. As the antioxidant, ascorbic acid or its salt may be used singly or, preferably, in combination of ascorbic acid or its salt with another antioxidant. As preferable examples, there may be mentioned a combination of ascorbic acid with sodium pyrosulfite. The antioxidant is added so as to be contained in a concentration of 0.005 to 1 w/v%, preferably 0.01 to 0.5 w/v%.

The preparation of the present invention may further contain a saccharide. Examples of the saccharide include: monosaccharidessuch asglucose,galactose,ribose,xylose,mannose, maltotriose, and maltotetraose; disaccharides such as sucrose, lactose, cellobiose, and maltose; trisaccharides such as raffinose; sugar alcohols such as sorbitol, inositol, and mannitol; polysaccharides such as dextran, chondroitin sulfate, hyaluronic acid, and dextrin sulfate; and their salts. In the preparation of the present invention, use may be made of, as required, an osmotic pressure adjustor such as glucose, sodium chloride, potassium chloride, glycerin, or mannitol; a soothing agent such as glucose, benzyl alcohol, mepivacaine hydrochloride, xylocaine hydrochloride, procaine hydrochloride, carbocaine hydrochloride, glycerin, propylene glycol, or lidocaine hydrochloride; an antiseptic agent such as a p-hydroxybenzoic acid ester (for example, methyl p-hydroxybenzoate, ethyl p-hydroxybenzoate or propyl p-hydroxybenzoate), thimerosal, chlorobutanol, or benzyl alcohol, or the like. The addition amounts of these substances can easily be chosen by a person skilled in the art according to techniques known per se.

In the preparation of the present invention, a light stabilizer may further be added, if desired. A pharmacologically acceptable light stabilizer may be used widely, and the examples include: sugars such as glucose, sucrose, fructose, and maltose; sugar alcohols such as xylitol, sorbitol, and mannitol; and polyalcohols such as glycerin and propylene glycol. In addition, as other preferable light stabilizers, use may be made of benzotriazole derivatives, benzophenone derivatives, salicylic acid derivatives, p-aminobenzoic acid derivatives, cinnamic acid derivatives, urocanic acid derivatives, riboflavin, folic acid, and so forth. One or two or more of these light stabilizers may be added to the preparation of the present invention. The light stabilizers may be added so as to be contained in a concentration of 0.01 to 1 w/v%, preferably 0.05 to 0.1 w/v%.

If necessary, the preparation of the present invention may contain another drug (e.g., an anticancer drug such as 5-fluorouracil, uracil, or mitomycin). Alternatively, the other drug is separately packed, and there may be produced a kit product including the drug in combination with the preparation of the present invention.

In the present specification, the term "an aqueous preparation for injection" means not only an injectable solution in final form, but also an injection solution precursor, e.g., a liquid injectable solution (concentrated or concentrated injectable solution), which can be converted to an injection solution in a final form by adding a solvent(diluent).

The preparation of the present invention can be produced by methods known per se. For example, the pH value of an aqueous solution containing sodium chloride, tromethamine, ascorbic acid, and calcium levofolinate is adjusted to a predetermined value, and then the solution is diluted so as to have a predetermined concentration of calcium levofolinate. Thereafter, the solution is filtered and sterilized under an aseptic condition, and a container such as an ampule, vial, or syringe (prefilled syringe) is filled with the resultant filtrate and is sealed, thereby to yield an aqueous preparation of the present invention.

For the preparation of the present invention, at least one package form selected from the following is employed for maintaining better preservation stability.
1) Inclusion of the preparation of the present invention in a glass or plastic container
   The inclusion of the preparation of the present invention in a glass or plastic container is used to avoid air incorporation through the wall of the container during the preservation period in order to minimize decomposition of levofolinate caused by air. As the material of the glass or plastic container, use is made of a widely known material as long as it possesses air impermeability. In the case of a plastic material, it may be difficult to completely eliminate air permeability, and therefore it is preferable to use a plastic container together with the following additional means (2), (3).
2) Inclusion of the preparation of the present invention in an inner container in a double package form which comprises an inner container and an outer bag, where a deoxidant(oxygen absorber) is placed between the inner container and the outer bag
   Examples of deoxidants are iron-based such as AGELESS ZH (trade name) and AGELESS ZP-50 (trade name); organic compound-based such as AGELESS G (trade name) and AGELESS GL (trade name) (which are manufactured by Mitsubishi Gas Chemical Company, Inc. Japan); and catechol-based such as TAMOTSU A (trade name) and TAMOTSU D (trade name) (which are manufactured by Oj itac Co., Ltd. Japan). This double package form is used to accomplish better air impermeability, in the case where a container itself has incomplete air impermeability, and to prevent air permeability into the container by putting a deoxidant as mentioned above in the space between the container and an outer bag in a double package. In this case, use of an inner container made of polypropylene(PP), polyethylene(PE), ethylene-vinyl acetate copolymer (EVA) and so on enables air permeation from the container to the inside of the outer bag, while as the outer bag, use is made of a material having higher air impermeability, for example, a material having a high density, or a metal bag.
3) Inclusion of the preparation of the present invention in a container where air in the inside space of the container is substituted with an inert gas
   Decomposition caused by air (oxygen) can be prevented by means of substituting an inert gas such as nitrogen gas for air in the inside space of a container to be filled with the preparation of the present invention.
4) Inclusion of the preparation of the present invention in a glass or plastic prefilled syringe;
   A so-called prefilled syringe which has a syringe function and an aqueous solution-holding function is filled with the preparation of the present invention. In the case of a plastic material, it is preferable to use a plastic container together with the following additional means (5), (6).
5) Inclusion of the preparation of the present invention in a prefilled syringe as an inner container of a double package form which comprises an inner container and an outer bag, where a deoxidant is included between the inner container and the outer bag; and
6) Inclusion of the preparation of the present invention in a prefilled syringe where air in the inside of the prefilled syringe is substituted by an inert gas.

In the preparation of the present invention, the pH value of the aqueous preparation is most desirably 6 to 7, the antioxidant is a combination of ascorbic acid with sodium pyrosulfite, and the concentration of the antioxidant is 0.1 to 0.3 w/v%. However, in the case where the concentration of sodium pyrosulfite is not less than 0.2 w/v%, precipitants form in the aqueous preparation. The preparation of the present invention is desirably included in an inner container, e.g., a syringe in a double package form which comprises an inner container and an outer bag, where a deoxidant(oxygen absorber) is placed between the inner container and the outer bag. The preparation of the present invention can be used as a pharmaceutical. The content of calcium salt of 5-formyl-(6S)-tetrahydrofolic acid (i.e. calcium levofolinate) is 90% or more during storage of 40°C/4 months (this condition is equivalent to 25 °C/2 years).

A pH value of an aqueous solution containing the calcium salt of 5-formyl- (6S) -tetrahydrofolic acid which has been adjusted to pH 6 to 7 gradually changes to an acid region during storage. The change of the pH value to the acid region can effectively be suppressed by ascorbic acid, and is more effectively suppressed by a combination of ascorbic acid with sodium pyrosulfite.

A transmittance of an aqueous solution containing the calcium salt of 5-formyl-(6S)-tetrahydrofolic acid lowers gradually during storage, and the solution colors. The lowering of the transmittance and the coloring can effectively be suppressed by ascorbic acid, and is more effectively suppressed by a combination of ascorbic acid with sodium pyrosulfite. The suppressing effect can be attained by the action of ascorbic acid which has been stabilized by sodium pyrosulfite.

The lowering of the transmittance and the coloring can most effectively be suppressed by including the preparation of the present invention in an inner container in a double package form which comprises an inner container and an outer bag, where a deoxidant(oxygen absorber) is placed between the inner container and the outer bag

In the present invention, parenteral administration, particularly intravenous administration is generally used as an administration method of the preparation of the present invention. The dosage may be determined according to a conventionally known dosage and administration method. A procedure for using the preparation of an injection solution is exemplified as follows. A preparation including 5-formyl-(6S)-tetrahydrofolic acid or a pharmacologically acceptable salt thereof, for example, 2 to 30 ml of a liquid formulation containing 10 to 100 mg of 5-formyl-(6S)-tetrahydrofolic acid or a pharmacologically acceptable salt thereof, 5 to 100 mg of an antioxidant such as ascorbic acid as, and 10 to 50 mg of a pH regulator such as tromethamine, is optionally diluted with a solution such as saline, a 5% aqueous glucose solution, or an electrolyte-containing solution, and the resultant solution containing 0.01 to 0.1 w/v% of 5-formyl-(6S)-tetrahydrofolic acid or a pharmacologically acceptable salt thereof is intravenously administered by infusion. In this case, the preparation is administered at an infusion rate of 0.5 to 30 µg/minute per 1 kg of body weight. The administration is performed once to three times a day concomitantly with administration of a fluorouracil agent to enhance an antitumor effect in fluorouracil therapy.

Hereinafter, the present invention will be described in detail by way of examples. In the Examples, "RH" means relative humidity.

### Example 1 Stabilizing effect by antioxidant

### (1) A preparation containing ascorbic acid

25 ml of water for injection were added to 0.224 g of sodium chloride and 0.550 g of calcium levofolinate, and the components were dissolved under heating to 45 to 50°C. To the resultant solution were added 0.12 g of tromethamine and 0.04 g of ascorbic acid, with stirring and bubbling of nitrogen gas, and then the total volume of the solution was adjusted to 30 ml by adding water for injection. Thereafter, 5% (weight /volume%, hereinafter, the term has the same meaning) hydrochloric acid was dropwise added to adjust the pH value of the solution to 8.1. Then the total volume of the solution was adjusted to 40 ml by adding water for injection.

### (2) A preparation containing sodium pyrosulfite

25 ml of water for injection were added to 0.224 g of sodium chloride and 0.550 g of calcium levofolinate, and the components were dissolved under heating to 45 to 50°C. To the resultant solution were added 0.12 g of tromethamine and 0. 008 g of sodium pyrosulfite, with stirring and bubbling of nitrogen gas, and then the total volume of the solution was adjusted to 30 ml by adding water for injection. Thereafter, 5% hydrochloric acid was dropwise added to adjust the pH value of the solution to 8.1, and then the total volume of the solution was adjusted to 40 ml by adding water for injection.

### (3) A preparation containing thioglycerol

25 ml of water for injection were added to 0.224 g of sodium chloride and 0.550 g of calcium levofolinate, and the components were dissolved under heating to 45 to 50°C. To the resultant solution were added 0.12 g of tromethamine and 0.08 g of thioglycerol, with stirring and bubbling of nitrogen gas, and then the total volume of the solution was adjusted to 30 ml by adding water for injection. Thereafter, 5% hydrochloric acid was dropwise added to adjust the pH value of the solution to 8.1, and then the total volume of the solution was adjusted to 40 ml by adding water for injection.

### (4) A preparation containing cysteine hydrochloride

50 ml of water for injection were added to 0.448 g of sodium chloride and 1.100 g of calcium levofolinate, and the components were dissolved under heating to 45 to 50°C. To the resultant solution were added 0.24 g of tromethamine, with stirring and bubbling of nitrogen gas, and then the total volume of the solution was adjusted to 60 ml by adding water for injection. To 30 ml of the thus-obtained solution were added 0.09 g of cysteine hydrochloride, and 5% hydrochloric acid was dropwise added to adjust the pH value of the solution to 8.1, and then the total volume of the solution was adjusted to 40 ml by adding water for injection.

### (5) A preparation containing thioglycolic acid

In the procedure of item (4) above, 0.09 g of thioglycolic acid was added instead of 0.09 g of cysteine hydrochloride, and 1% aqueous sodium hydroxide solution was used instead of 5% hydrochloric acid.

### (6) A preparation containing sodium hydrogensulfite

25 ml of water for injection was added to 0.224 g of sodium chloride and 0.550 g of calcium levofolinate, and the components were dissolved under heating to 45 to 50°C. To the resultant solution were added 0.18 g of glycine, with stirring and bubbling of nitrogen gas, followed by adjusting the pH value of the solution to 7.0 by adding 5% hydrochloric acid dropwise.

After adding 0.008g of sodium hydrogensulfite with stirring and bubbling of nitrogen gas, the total volume of the solution was adjusted to 30 ml by adding water for injection. Thereafter, 1% aqueous sodium hydroxide solution was dropwise added to adjust the pH value of the solution to 7.0, and then the total volume of the solution was adjusted to 40 ml by adding water for injection.

### (7) A preparation containing sodium sulfite

25 ml of water for injection were added to 0.224 g of sodium chloride and 0.550 g of calcium levofolinate, and the components were dissolved under heating to 45 to 50°C. To the resultant solution were added 0.12 g of tromethamine and 0.012 g of sodium sulfite, with stirring and bubbling of nitrogen gas, and then the total volume of the solution was adjusted to 30 ml by adding water for injection. Thereafter, 5% hydrochloric acid solution was dropwise added to adjust the pH value of the solution to 7.0, and then the total volume of the solution was adjusted to 40 ml by adding water for injection.

The aqueous preparations obtained in (1) to (7) above were separately filtered with a filtration filter (pore size: 0.2 µm, manufactured by Millipore Corporation, hereinafter, the term has the same meaning). Subsequently, each of seven 5 ml-glass ampules where air in the inside space of the ampule had been substituted by nitrogen gas was filled with 5 ml of one of the filtrates, and was sealed by heating, thereby to produce an ampules containing an aqueous preparation for injection.

### Stability Test

For the aqueous preparations obtained in (1) to (3) above, tests evaluating stability at 60°C/3 weeks were performed. The calcium levofolinate content remaining in the aqueous solution was measured using high performance liquid chromatography under the following conditions (the same were employed in the following experiments). 2 ml each of the test preparations were weighed, and 10 ml of the internal standard solution described below (the same was employed in the following experiments) were added thereto. Subsequently, water was added to adjust the total volume of the solution to 50 ml, thereby to prepare a test solution. Meanwhile, 62.5mg of calcium levofolinate were dissolved in water so as to prepare a 25 ml solution. Then, to 10 ml of the resultant solution were added 10 ml of the internal standard solution, and the total volume of the solution was adjusted to 50 ml by adding water, thereby to prepare a standard solution. For 20 µl of the standard solution and the test solution, the content of calcium levofolinate was measured by the liquid chromatography method under the following conditions.

### 1) Measurement conditions

Detector: Ultraviolet absorptiometer (Measurement wavelength: 254 nm)
Column: A stainless tube having an internal diameter of 4.0 mm and a length of 15 cm was filled with 5 µm of octadecylsilylated silica gel for liquid chromatography.
Column temperature: A constant temperature near 45°C.
Mobile phase: The pH value of a mixed test solution containing 0. 008 mol/L of disodium hydrogen phosphate solution/methanol/ 40% aqueous tetrabutylammonium hydroxide solution (385 : 110 : 1.3 (volume ratio)) was adjusted to 7.5 by adding phosphoric acid.
Flow rate: The flow rate was regulated so that the retention time of calcium levofolinate became about 10 minutes.
Internal standard solution: A solution prepared by adding water to 0.04 g of methyl parahydroxybenzoate so that the whole amount of solution is 100 ml.

### 2) Results

The results are shown in Table 1-1. Table 1-1 shows variations compared with the initial value of calcium levofolinate (100). The stabilizing effect by adding an antioxidant was confirmed, particularly, an extremely excellent stabilizing effect was confirmed in the case of addition of ascorbic acid. The stabilizing effect by each antioxidant was analyzed by the high performance liquid chromatography method. As a result, the stabilizing effect of each stabilizer was confirmed to show significant specificity.

**Table 1-1**

| Antioxidant species | | | | | |
|---|---|---|---|---|---|
| Sample No. | Antioxidant | 60°C | | | |
| | | Initial value | 1 week | 2 weeks | 3 weeks |
| (1) | Ascorbic acid | 100 | 97.4 | 93.1 | 87.7 |
| | | | | | |
| (2) | Sodium pyrosulfite | 100 | 95.5 | 92.6 | 87.6 |
| | | | | | |
| (3) | Thioglycerol | 100 | 94.9 | 90.3 | 86.3 |
| | | | | | |

For the aqueous preparations obtained in (4) to (7) above, tests evaluating stability at 60°C/2 weeks were performed in a manner similar to the above. The results are shown in Table 1-2. The stabilizing effect of each antioxidant was analyzed by the high performance liquid chromatography method. As a result, the stabilizing effect of each stabilizer was confirmed to show significant specificity.

**Table 1-2**

| Antioxidant species | | | | |
|---|---|---|---|---|
| Sample No. | Antioxidant | 60°C | | |
| | | Initial value | 1 week | 2 weeks |
| (4) | Cysteine hydrochloride | 100 | 94.2 | 86.4 |
| | | | | |
| (5) | Thioglycolic acid | 100 | 96.1 | 92.7 |
| | | | | |
| (6) | Sodium hydrogensulfite | 100 | 93.4 | 92.0 |
| | | | | |
| (7) | Sodium sulfite | 100 | 95.0 | 94.7 |
| | | | | |

### Example 2 Study on effect of pH value (addition of ascorbic acid)

About 80 ml of water for injection were added to a beaker (volume: 200 ml). Then 0.56 g of sodium chloride, 0.3 g of tromethamine, and 0. 5 g of ascorbic acid were added to it with bubbling of nitrogen gas, and the components were dissolved with stirring. Subsequently, to the resultant solution were added 1.375 g of calcium levofolinate (1.1 g, in terms of 5-formyl-(6S)-tetrahydrofolic acid), and the components were dissolved with stirring under heating to 45 to 50°C. Thereafter, the pH value of the solution was adjusted to 5.0 by adding 5% hydrochloric acid solution dropwise. A similar procedure to the above, except using 1% aqueous sodium hydroxide solution in place of 5% hydrochloric acid solution, was performed to produce solutions, each having a regulated pH value of 6.0, 7.0, 8.0, or 9.0. Water for injection was further added to each solution to adjust the total volume of the solution to 100 ml. Subsequently, the bubbling of nitrogen gas was stopped, and each solution was filtered with a filtration filter, thereby to yield a solution for injection. Subsequently, each of five 5 ml-glass ampules the inside space of which had been substituted by nitrogen gas, was filled with 5 ml of the resultant solution for injection, and was sealed by heating.

For each aqueous preparation obtained as above, a stability test at 60°C was performed by measuring the remaining calcium levofolinate content by the high performance liquid chromatography method. Table 2 shows variations compared with the initial value of calcium levofolinate (100). In the cases of a pH of about 6 to 7, an extremely excellent stabilizing effect was demonstrated.

**Table 2**

| Effect of pH value (Addition of ascorbic acid) | | | | |
|---|---|---|---|---|
| pH value | 60°C | | | |
| | Initial value | 1 week | 2 weeks | 3 weeks |
| 5.0 | 100 | 91.5 | 87.5 | 86.0 |
| | | | | |
| 6.0 | 100 | 95.8 | 94.1 | 92.8 |
| | | | | |
| 7.0 | 100 | 96.3 | 93.4 | 92.5 |
| | | | | |
| 8.0 | 100 | 94.4 | 89.2 | 87.0 |
| | | | | |
| 9.0 | 100 | 95.7 | 89.7 | 85.3 |
| | | | | |

### Example 3 Study on effect of pH value (no addition of ascorbic acid)

About 80 ml of water for injection were added to a beaker (volume: 200 ml). Then, 0.56 g of sodium chloride and 0.3 g of tromethamine were added to it with bubbling of nitrogen gas, and the components were dissolved with stirring and heating to 45 to 50 °C. Subsequently, 1.375 g of calcium levofolinate (1.1 g, in terms of 5-formyl-(6S)-tetrahydrofolic acid) were added, and the components were dissolved with stirring. Thereafter, the pH value of the solution was adjusted to 6. 0 by adding 5% hydrochloric acid solution dropwise. A similar procedure to the above was performed to produce solutions, each having a regulated pH value of 7.0, 7.5, 7.9, 8.1, 8.3, 8.7, 9.0, 9.2, and 9. 5 (1% aqueous sodium hydroxide solution was used in place of 5% hydrochloric acid solution).

Water for injection was further added to each solution to adjust the total volume of the solution to 100 ml. Subsequently, the bubbling of nitrogen gas was stopped, and each solution was filtered with a filtration filter, thereby to yield a solution for injection. Subsequently, each of nine 5 ml-glass ampules the inside space of which had been substituted by nitrogen gas was filled with 5 ml of the resultant solution for injection, and was sealed by heating.

For each aqueous preparation obtained as above, a test evaluating stability at 60°C was performed by measuring the remaining levofolinate content by the high performance liquid chromatography method. Table 3 shows variations compared with the initial value of calcium levofolinate (100). In the cases of pH 6 to 7 and pH 8.7 to pH 9, an extremely excellent stabilizing effect was confirmed.

**Table 3**

| Effect of pH value (no addition of ascorbic acid) | | | | | |
|---|---|---|---|---|---|
| pH value | 60°C | | | | |
| | Initial value | 1 week | 2 weeks | 3 weeks | 1 month |
| | | | | | |
| 6.0 | 100 | 94.7 | 92.5 | 90.1 | 88.7 |
| | | | | | |
| 7.0 | 100 | 94.8 | 92.2 | 90.1 | 88.6 |
| | | | | | |
| 7.5 | 100 | 93.4 | 89.0 | 86.8 | 84.2 |
| | | | | | |
| 7.9 | 100 | 95.1 | 89.7 | 86.3 | 81.9 |
| | | | | | |
| 8.1 | 100 | 95.4 | 91.5 | 85.6 | - |
| | | | | | |
| 8.3 | 100 | 93.6 | 91.1 | 85.5 | - |
| | | | | | |
| 8.7 | 100 | 94.7 | 93.6 | 88.3 | - |
| | | | | | |
| 9.0 | 100 | 96.1 | 93.3 | 88.6 | - |
| | | | | | |
| 9.2 | 100 | 94.4 | 93.4 | 87.7 | - |
| | | | | | |
| 9.5 | 100 | 93.7 | 92.4 | 85.3 | - |
| | | | | | |

### Example 4 Study on addition amount of ascorbic acid

About 80 ml of water for injection were added to a beaker (volume: 200 ml). Then 0.56 g of sodium chloride, 0.3 g of tromethamine, and 1. 375 g of calcium levofolinate (1.1 g, in terms of 5-formyl-(6S)-tetrahydrofolic acid) were added to it with bubbling of nitrogen gas, and the components were dissolved with stirring (this procedure was performed five times). Subsequently, 0 (no addition), 0.05 g, 0.1 g, 0.3 g, 0.5 g or 1.0 g of ascorbic acid was added to each solution, and was dissolved with stirring, to prepare six solutions. Thereafter, in the case where the addition amount of ascorbic acid is 0 (no addition), 0.05 g, or 0.1 g, the pH value of each solution was adjusted to 8.1 by adding 5% hydrochloric acid dropwise. In the case where the addition amount of ascorbic acid is 0.3 g, 0.5g, or 1.0 g, the pH value of each solution was adjusted to 8.1 by adding 1% aqueous sodium hydroxide solution dropwise. Water for injection was further added to each solution to adjust the total volume of the solution to 100 ml. Subsequently, the bubbling of nitrogen gas was stopped, and each solution was filtered with a filtration filter, thereby to yield a solution for injection. Subsequently, each of five 5 ml-glass ampules the inside space of which had been substituted by nitrogen gas, was filled with 5 ml of the resultant solution for injection, and it was sealed by heating.

For each aqueous preparation obtained as above, a test evaluating stability at 60°C was performed by measuring the remaining calcium levofolinate content by the high performance liquid chromatography method. Table 4 shows variations compared with the initial value of calcium levofolinate (100).

**Table 4**

| Addition amount of ascorbic acid /pH 8.1 | | | | | |
|---|---|---|---|---|---|
| Addition amount of Ascorbic acid | Initial value | 60°C | | | |
| | | 1 week | 2 weeks | 3 weeks | 1 month |
| 0% (No addition) | 100 | 94.1 | 88.7 | 86.0 | 79.7 |
| 0.05% | 100 | 96.4 | 92.4 | 87.3 | 82.6 |
| 0.1% | 100 | 97.4 | 93.1 | 87.7 | 82.2 |
| 0.3% | 100 | 96.3 | 91.3 | 86.9 | 81.6 |
| 0.5% | 100 | 96.5 | 90.9 | 86.0 | 80.9 |
| 1.0% | 100 | 95.4 | 89.8 | 84.1 | 80.3 |

### Example 5 Study on the addition amount of ascorbic acid

About 80 ml of water for injection were taken in a beaker (volume: 200 ml). Then, 0.56 g of sodium chloride, 0.3 g of tromethamine, and 1.375 g of calcium levofolinate (1.1 g, in terms of 5-formyl- (6S) -tetrahydrofolic acid) were added to it with bubbling of nitrogen gas, and the components were dissolved with stirring (this procedure was performed 5 times). Subsequently, 0. 05 g, 0.075 g, 0.1g, 0. 2 g, or 0.3g of ascorbic acid were added to each solution, and was dissolved with stirring, to prepare five solutions. Thereafter, in the case where the addition amount of ascorbic acid is 0.05 g, 0.075 g, or 0.1 g, the pH value of each solution was regulated to 8.7 by adding 5% hydrochloric acid. In the case where the addition amount of ascorbic acid is 0.2 g, or 0.3 g, the pH value of each solution was regulated to 8.7 by adding 1% aqueous sodium hydroxide solution dropwise. Water for injection was further added to each solution to adjust the total volume of the solution to 100 ml. Subsequently, the bubbling of nitrogen gas was stopped, and each solution was filtered with a filtration filter, thereby to yield a solution for injection. Subsequently, each of five 5 ml-glass ampules the inside space of which had been substituted by nitrogen gas, was filled with 5 ml of the resultant solution for injection, and was sealed by heating.

For each aqueous preparation obtained as above, a test evaluating stability at 60°C was performed by measuring the remaining calcium levofolinate content by the high performance liquid chromatography method. Table 5 shows variations compared with the initial value of calcium levofolinate (100).

**Table 5 Addition amount of ascorbic acid /pH 8.7**

| Addition amount of Ascorbic acid | Initial value | 60°C | | | |
|---|---|---|---|---|---|
| | | 1 week | 2 weeks | 3 weeks | 1 month |
| 0.05% | 100 | 97.0 | 92.1 | 90.3 | 84.5 |
| 0.075 % | 100 | 97.1 | 91.5 | 90.9 | 85.7 |
| 0.1% | 100 | 97.3 | 92.2 | 89.3 | 84.9 |
| 0.2% | 100 | 96.6 | 91.5 | 88.6 | 83.4 |
| 0.3% | 100 | 96.1 | 90.5 | 87.7 | 82.0 |

### Example 6 Addition of a plurality of antioxidants

About 1100 ml of water for injection were added to a beaker. Then, 8.04 g of sodium chloride, 3.6 g of tromethamine, 3.6 g of ascorbic acid, and 1.2 g of sodium pyrosulfite were added thereto with bubbling of nitrogen gas, and the components were dissolved with stirring. To the resultant solution were added 15.62 g of calciumlevofolinate,which weredissolved withstirring.Thereafter, the pH value of the solution was adjusted to 6.2 by adding 5% hydrochloric acid solution dropwise. Water for injection was further added to the solution to adjust the total volume to 1200 ml. Subsequently, the bubbling of nitrogen gas was stopped, and the solution was filtered with a filtration filter, thereby to yield a solution for injection. A 5 ml-syringe made of polypropylene, the inside space of which had been substituted by nitrogen gas, was filled with 5 ml of the resultant solution for injection, followed by steam sterilization under high pressure at 105°C for 30 minutes. The thus-sterilized syringe was packaged together with a deoxidant, AGELESS ZP-50(trade name, Mitsubishi Gas Chemical Company, Inc. Japan) by using a plastic film with high air impermeability.

For an aqueous preparation as obtained above, a stability test (60°C/75%RH, and 40°C/75%RH) was performed by measuring the content of calcium levofolinate remaining after storage for a certain period by the high performance liquid chromatography method.

Table 6 shows ratio(%) of remaining calcium levofolinate compared with the initial value of calcium levofolinate (100).

**Table 6**

| Addition of ascorbic acid and sodium pyrosufite | | |
|---|---|---|
| Storage condition | Storage period | remaining ratio(%) |
| 60°C/75%RH | Initial value | 100 |
| | 1 week | 99.0 |
| | 2 weeks | 97.5 |
| | 3 weeks | 95.8 |
| | 4 weeks | 94.3 |
| 40°C/75%RH | Initial value | 100 |
| | 1 month | 101.2 |
| | 2 months | 100.2 |
| | 4 months | 95.7 |
| | 6 months | 92.8 |

### Example 7

About 1100 ml of water for injection were added to a beaker. Then, 8.04 g of sodium chloride, 3.6 g of tromethamine, and 1.2 g of ascorbic acid were added thereto with bubbling of nitrogen gas, and the components were dissolved with stirring. To the resultant solution were added 15. 62 g of calcium levofolinate, which was dissolved with stirring. Thereafter, the pH value of the solution was adjusted to 6.2 by adding 5% hydrochloric acid solution dropwise. Water for injection was further added to the solution to adjust the total volume to 1200 ml. Subsequently, the bubbling of nitrogen gas was stopped, and the solution was filtered with a filtration filter, thereby to yield a solution for injection. A 5 ml-syringe made of polypropylene, the inside space of which had been substituted by nitrogen gas, was filled with 5 ml of the resultant solution for injection, followed by steam sterilization under high pressure at 105°C for 30 minutes. The thus-sterilized syringe was packaged together with a deoxidant, AGELESS ZP-50(trade name, Mitsubishi Gas Chemical Company, Inc. Japan) by using a plastic film with high air impermeability.

For an aqueous preparation as obtained above, a stability test (60°C/75%RH, and 40°C/75%RH) was performed by measuring the content of calcium levofolinate remaining after storage for a certain period by the high performance liquid chromatography method.

Table 7 shows ratio(%) of remaining calcium levofolinate compared with the initial value of calcium levofolinate (100).

**Table 7**

| Addition of 0.1% ascorbic acid | | |
|---|---|---|
| Storage condition | Storage period | remaining ratio(%) |
| | Initial value | 100 |
| | 1 week | 96.6 |
| 60°C/75%RH | 2 weeks | 96.2 |
| | 3 weeks | 95.3 |
| | 4 weeks | 94.4 |
| | Initial value | 100 |
| | 1 month | 99.8 |
| 40°C/75%RH | 2 months | 98.8 |
| | 4 months | 96.6 |
| | 6 months | 91.8 |

The aqueous preparation for injection containing 5-formyl-(6S)-tetrahydrofolic acid or a pharmacologically acceptable salt thereof is extremely stable for a long time, and thus the present invention provides a new industrial application of 5-formyl-(6S)-tetrahydrofolic acid or a pharmacologically acceptable salt.

## Claims

1. An aqueous preparation for injection consisting of 5-formyl-(6S)-tetrahydrofolic acid or a pharmacologically acceptable salt thereof as active ingredient, a basification material or a buffer agent as pH regulator, and antioxidant selected from the group consisting of ascorbic acid or a salt thereof, sodium pyrosulfite, cysteine hydrochloride, sodium hydrogensulfite, sodium sulfite and thioglycolic acid or a salt thereof;
and optionally further consisting of a saccharide, an osmotic pressure adjustor, a soothing agent, an antiseptic agent, a light stabilizer, and another anticancer drug selected from the group consisting of 5-fluorouracil, uracil or mitomycin, wherein an adjusted pH value of the aqueous solution is 6 to 7.

2. The preparation according to claim 1, wherein the pharmacologically acceptable salt of 5-formyl-(6S)-tetrahydrofolic acid is an alkali metal salt or an alkaline earth metal salt.

3. The preparation according to claim 1, wherein the pharmacologically acceptable salt of 5-formyl-(6S)-tetrahydrofolic acid is a calcium salt.

4. The preparation according to any one of claims 1 to 3, wherein the basification material is tromethamine.

5. The preparation according to any one of claims 1 to 3, wherein the buffer agent is glycine.

6. The preparation according to any one of claims 1 to 5, wherein the antioxidant is ascorbic acid or a salt thereof.

7. The preparation according to any one of claims 1 to 5, wherein the antioxidant is a combination of ascorbic acid or a salt thereof with another antioxidant.

8. The preparation according to any one of claims 1 to 7, wherein the antioxidant is present in an amount of 0.005 to 1 w/v%.

9. The preparation according to any one of claims 4 to 7, wherein tromethamine or glycine is mixed in an amount of 0.01 to 1 w/v%.

10. A pharmaceutical package comprising the preparation defined in claim 1, contained in at least one package form selected from:
1) a glass or plastic container;
2) an inner container of a double package form which comprises an inner container and an outer bag, where a deoxidant is included between the inner container and the outer bag;
3) a container where air in the inside of the container is substituted by an inert gas;
4) a glass or plastic prefilled syringe;
5) a prefilled syringe as an inner container of a double package form which comprises an inner container and an outer bag, where a deoxidant is included between the inner container and the outer bag; and
6) a prefilled syringe where air in the inside of the prefilled syringe is substituted by an inert gas.

11. A method of stabilizing an aqueous preparation for injection according to claim 1 , wherein antioxidant selected from the group consisting of ascorbic acid or a salt thereof, sodium pyrosulfite, cysteine hydrochloride, sodium hydrogensulfite, sodium sulfite and thioglycolic acid or a salt thereof is added to an aqueous solution of 5-formyl-(6S)-tetrahydrofolic acid or a pharmacologically acceptable salt thereof as active ingredient, wherein tromethamine or glycine is further added, and wherein an adjusted pH value of the aqueous solution is 6 to 7.

12. A pharmaceutical aqueous preparation for injection according to claim 1, which consists of calcium levofolinate as an active ingredient, ascorbic acid or a salt thereof as antioxidant, and tromethamine or glycine as pH regulator.

## Patentansprüche

1. Wässrige Zubereitung zur Injektion, bestehend aus 5-Formyl-(6S)-tetrahydrofolsäure oder einem pharmakologisch akzeptablen Salz davon als aktivem Ingrediens, mit einem Basifizierungsmaterial oder einem Puffermittel als pH-Regulator und Antioxidans, ausgewählt aus der Gruppe, bestehend aus Ascorbinsäure oder einem Salz davon, Natriumpyrosulfit, Cystein-Hydrochlorid, Natriumhydrogensulfit, Natriumsulfit und Thioglycolsäure oder einem Salz davon;
und gegebenenfalls außerdem bestehend aus einem Saccharid, einem Einstellmittel für den osmotischen Druck, einem Beruhigungsmittel, einem Antiseptikum, einem Lichtstabilisator und einem anderen Antikrebsarzneimittel, ausgewählt aus der Gruppe, bestehend aus 5-Fluorouracil, Uracil oder Mitomycin, wobei der eingestellte pH-Wert der wässrigen Lösung 6 bis 7 ist.

2. Zubereitung nach Anspruch 1, wobei das pharmakologisch verträgliche Salz von 5-Formyl-(6S)-tetrahydrofolsäure ein Alkalimetallsalz oder ein Erdalkalimetallsalz ist.

3. Zubereitung nach Anspruch 1, wobei das pharmakologisch verträgliche Salz von 5-Formyl-(6S)-tetrahydrofolsäure ein Calciumsalz ist.

4. Zubereitung nach einem der Ansprüche 1 bis 3, wobei das Basifizierungsmaterial Tromethamin ist.

5. Zubereitung nach einem der Ansprüche 1 bis 3, wobei das Puffermittel Glycin ist.

6. Zubereitung nach einem der Ansprüche 1 bis 5, wobei das Antioxidans Ascorbinsäure oder ein Salz davon ist.

7. Zubereitung nach einem der Ansprüche 1 bis 5, wobei das Antioxidans eine Kombination von Ascorbinsäure oder einem Salz davon mit einem anderen Antioxidans ist.

8. Zubereitung nach einem der Ansprüche 1 bis 7, wobei das Antioxidans in einer Menge von 0,005 bis 1 G/V-% vorliegt.

9. Zubereitung nach einem der Ansprüche 4 bis 7, wobei Tromethamin oder Glycin in einer Menge von 0,01 bis 1 G/V-% zugemischt ist.

10. Pharmazeutische Packung, umfassend die Zubereitung nach Anspruch 1, die in wenigstens einer Packungsform enthalten ist, welche ausgewählt ist aus:
1) einem Glas- oder Kunststoffbehälter;
2) einem inneren Behälter einer Doppelpackungsform, welche einen inneren Behälter und einen äußeren Beutel umfasst, wobei zwischen dem inneren Behälter und dem äußeren Beutel ein Desoxidationsmittel enthalten ist;
3) einem Behälter, in dem Luft im Inneren des Behälters durch ein Inertgas ersetzt ist;
4) einer vorgefüllten Glas- oder Kunststoffspritze;
5) einer vorgefüllten Spritze als innerer Behälter einer Doppelpackungsform, welche einen inneren Behälter und einen äußeren Beutel umfasst, wobei ein Desoxidationsmittel zwischen dem inneren Behälter und dem äußeren Beutel enthalten ist, und
6) einer vorgefüllten Spritze, in der Luft im Inneren der vorgefüllten Spritze durch ein Inertgas ersetzt ist.

11. Verfahren zum Stabilisieren einer wässrigen Zubereitung zur Injektion gemäß Anspruch 1, wobei ein Antioxidans, ausgewählt aus der Gruppe, bestehend aus Ascorbinsäure oder einem Salz davon, Natriumpyrosulfit, Cystein-Hydroclorid, Natriumhydrogensulfit, Natriumsulfit und Thioglycolsäure oder einem Salz davon, zu einer wässrigen Lösung von 5-Formyl-(6S)-tetrahydrofolsäure oder einem pharmakologisch verträglichen Salz davon als aktives Ingrediens gegeben wird, wobei außerdem Tromethanin oder Glycin zugesetzt wird und wobei der eingestellte pH-Wert der wässrigen Lösung 6 bis 7 ist.

12. Pharmazeutische wässrige Zubereitung zur Injektion nach Anspruch 1, welche aus Calcium-Levofolinat als aktivem Ingrediens, Ascorbinsäure oder einem Salz davon als Antioxidans und Tromethamin oder Glycin als pH-Regulator besteht.

## Revendications

1. Préparation aqueuse pour injection composée d'acide 5-formyl-(6S)-tétrahydrofolique ou d'un sel de celui-ci acceptable sur le plan pharmacologique en tant qu'ingrédient actif, d'un matériau de basification ou d'un agent tampon en tant que régulateur de pH, et d'un antioxydant sélectionné dans le groupe composé de l'acide ascorbique ou d'un sel de celui-ci, du pyrosulfite de sodium, du chlorhydrate de cystéine, de l'hydrogénosulfite de sodium, du sulfite de sodium, et de l'acide thioglycolique ou d'un sel de celui-ci ;
et en option composée en outre d'un saccharide, d'un agent d'ajustement de la pression osmotique, d'un agent calmant, d'un agent antiseptique, d'un agent photostabilisant, et d'un autre médicament anticancéreux sélectionné dans le groupe composé du 5-fluorouracile, de l'uracile et de la mitomycine, où une valeur ajustée du pH de la solution aqueuse est comprise entre 6 et 7.

2. Préparation selon la revendication 1, dans laquelle le sel acceptable sur le plan pharmacologique de l'acide 5-formyl-(6S)-tétrahydrofolique est un sel de métal alcalin ou un sel de métal alcalinoterreux.

3. Préparation selon la revendication 1, dans laquelle le sel acceptable sur le plan pharmacologique de l'acide 5-formyl-(6S)-tétrahydrofolique est un sel de calcium.

4. Préparation selon l'une quelconque des revendications 1 à 3, dans laquelle le matériau de basification est la trométhamine.

5. Préparation selon l'une quelconque des revendications 1 à 3, dans laquelle l'agent tampon est la glycine.

6. Préparation selon l'une quelconque des revendications 1 à 5, dans laquelle l'agent antioxydant est l'acide ascorbique ou un sel de celui-ci.

7. Préparation selon l'une quelconque des revendications 1 à 5, dans laquelle l'agent antioxydant est une combinaison d'acide ascorbique ou d'un sel de celui-ci avec un autre agent antioxydant.

8. Préparation selon l'une quelconque des revendications 1 à 7, dans laquelle l'agent antioxydant est présent dans une quantité comprise entre 0,005 et 1 % en poids/volume.

9. Préparation selon l'une quelconque des revendications 4 à 7, dans laquelle la trométhamine ou la glycine est mélangée dans une quantité comprise entre 0,01 et 1 % en poids/volume.

10. Ensemble pharmaceutique comprenant la préparation définie dans la revendication 1, contenue dans au moins une forme de conditionnement sélectionnée parmi les formes suivantes :
1) un conteneur en verre ou plastique ;
2) un conteneur interne d'une forme à double conditionnement qui comprend un conteneur interne et un sac externe, où un désoxydant est inclus entre le conteneur interne et le sac externe ;
3) un conteneur dans lequel l'air à l'intérieur du conteneur est substitué par un gaz inerte ;
4) une seringue de verre ou de plastique pré-remplie ;
5) une seringue pré-remplie en tant que conteneur interne d'une forme à double conditionnement qui comprend un conteneur interne et un sac externe, où un agent désoxydant est inclus entre le conteneur interne et le sac externe ; et
6) une seringue pré-remplie dans laquelle l'air à l'intérieur de la seringue pré-remplie est substitué par un gaz inerte.

11. Procédé de stabilisation d'une préparation aqueuse pour injection selon la revendication 1, dans lequel l'antioxydant sélectionné dans le groupe composé de l'acide ascorbique ou d'un sel de celui-ci, du pyrosulfite de sodium, du chlorhydrate de cystéine, de l'hydrogénosulfite de sodium, du sulfite de sodium et de l'acide thioglycolique ou d'un sel de celui-ci est ajouté à une solution aqueuse d'acide 5-formyl-(6S)-tétrahydrofolique ou d'un sel de celui-ci acceptable sur le plan pharmacologique en tant qu'ingrédient actif, où de la trométhamine ou de la glycine est en outre ajoutée, et où une valeur de pH ajustée de la solution aqueuse est comprise entre 6 et 7.

12. Préparation pharmaceutique aqueuse pour injection selon la revendication 1, qui se compose de lévofolinate de calcium en tant qu'ingrédient actif, d'acide ascorbique ou d'un sel de celui-ci en tant qu'antioxydant, et de trométhamine ou de glycine en tant que régulateur de pH.
